(19) **Europäisches Patentamt / European Patent Office / Office européen des brevets**

(11) **EP 4 407 314 A1**

(12) # EUROPEAN PATENT APPLICATION
published in accordance with Art. 153(4) EPC

(43) Date of publication:
**31.07.2024 Bulletin 2024/31**

(21) Application number: **22872209.6**

(22) Date of filing: **26.09.2022**

(51) International Patent Classification (IPC):
**G01N 33/483** (2006.01)   **G01N 21/55** (2014.01)

(52) Cooperative Patent Classification (CPC):
**G01N 21/01; G01N 21/55; G01N 21/552;
G01N 21/84; G01N 33/483**

(86) International application number:
**PCT/CN2022/121338**

(87) International publication number:
**WO 2023/046166 (30.03.2023 Gazette 2023/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.09.2021 CN 202111126875**

(71) Applicant: **Ruixin Fuzhou Technology Co., Ltd
Fuzhou, Fujian 350100 (CN)**

(72) Inventor: **LIN, Zhe
Fuzhou, Fujian 350100 (CN)**

(74) Representative: **Garavelli, Paolo
A.BRE.MAR. S.R.L.
Consulenza in Proprietà Industriale
Via Servais 27
10146 Torino (IT)**

(54) **CELLULAR MECHANICAL FORCE DETECTION SYSTEM, METHOD AND APPARATUS AND MANUFACTURING METHOD THEREFOR**

(57)    A cellular mechanical force detection apparatus (1), comprising: a base (11), and a micro-column array formed by a plurality of micro-columns (12) that are provided on the base (11) and can be deformed by means of the cellular mechanical force. The top of the micro-column (12) or the upper portion of the column surface has a light reflecting layer (13). Moreover, a detection system, a detection method, and a preparing method are provided. The solution has the advantages of high throughput, low cost, single cell resolution, real-time monitoring, high sensitivity, capability of simulating a cellular microenvironment, capability of simulating components and forms of extracellular matrixes, and the like, and can cope with richer technical demand scenarios.

FIG. 3

## Description

FIELD OF THE INVENTION

The present application relates to the field of biotechnology, in particular to a system, a method, a device and a preparing method thereof for detecting cellular mechanical force.

BACKGROUND OF THE INVENTION

Cells exert small cellular mechanical forces on their surrounding microenvironment. The cellular mechanical forces play a crucial role in processes including adhesion, migration, proliferation, differentiation and apoptosis. Combined with other biochemical signals, they are also vital for regulating embryonic development, stem cell differentiation, immune responses, wound healing, and cancer metastasis. Therefore, they are significant targets for disease treatment. Prior studies had shown substantial differences in the maximal cellular mechanical forces among normal, benign, and malignant tumor cells, making precise and high-throughput measurement of cellular mechanical forces to a core technology for many biomedical applications. However, the significance of measuring maximal cellular mechanical forces is limited. High-resolution, real-time, and high-throughput mechanical sensors will become the core requirements of the next generation of cell force measurement tools.

Current techniques for measuring cellular mechanical forces primarily include traction force microscopy (TFM), micro/nano cantilevers, and array of micropillars. These methods calculate cellular mechanical forces by measuring the deformation of an elastic substrate caused by cellular mechanical forces. So far, TFM has been the most widely used technique, with a principle based on a continuous elastic substrate (such as a wrinkled thin silicon film or a polyacrylamide (PA) gel embedded with fluorescent microbeads). When cells are cultured on an elastic substrate, the cells will apply cellular mechanical forces to the substrate to deform the substrate, and then the fluorescent microbeads will be displaced during the deformation process. Using fluorescence microscopy and other methods to track the trajectory of fluorescent microbeads, and image processing and other methods to get substrate strain information. When the substrate elasticity is known, the cellular mechanical forces can be deduced through a mechanical model.

However, this measurement method has the following disadvantages. First, TFM's reliance on fluorescent microscopy imaging and requires complex calculations. The process is complicated, the throughput is low, and the cost is high. Secondly, TFM does not directly measure the cellular mechanical forces but infers them from the displacement of fluorescent beads within the substrate. Although a special process can ensure that most of the particles are deposited on the surface, after a long time of immersion, they are very likely to escape or decline, leading to a decrease in the density of the surface fluorescent particles. Fluorescence microscopes usually have a large depth of field, which may capture particles in different planes, resulting in deviations in later displacement calculations and resulting in inaccurate measurement results. Additionally, a long-term immersion in the culture medium can alter the gel's elastic modulus, affecting the accuracy of cellular mechanical force calculations and necessitating modulus calibration, further increasing the workload. Further, a long-term laser exposure can also cause phototoxicity to cells and photoquenching of fluorescent microbeads, making TFM unsuitable for long-term, continuous detection of cellular mechanical forces. It usually takes a long time to study cell growth and differentiation as well as its response to drugs, this limitation significantly restricts TFM's application in the biomedical field.

Compared to TFM, micro-/nano-sensors, such as micro-cantilever arrays and array of micropillars, can also be used to directly measure cellular mechanical force. For example, in the array of micropillars, cells are attached to the micropillar upper surface, and the bending deformation of the micropillar can be calculated by photographing the base and top of the micropillar with a microscope, so as to infer the size and direction of the cellular mechanical force at this point. Similar to TFM, micro-sensors also rely on the high-resolution imaging of the microscope, which is very demanding on the imaging device, and it is easy to produce errors in the imaging process, resulting in inaccurate results. Furthermore, the obtained images necessitate complex image processing and mechanical model calculations to determine cellular mechanical forces, making the operation intricate, time-consuming, and challenging to achieve in real-time, high-throughput, low-cost, and long-term cellular monitoring. Thus, existing technologies are primarily confined to biomechanical research and pose challenges in practical applications.

SUMMARY OF THE INVENTION

Hence, there is a need for a quantitative measurement and monitoring solution for cellular mechanical force. This solution should address the limitations of existing technologies, be microscope-free, real-time, high-throughput, and cost-effective, thereby fulfilling the fundamental requirements of next-generation cell force measurement tools.

To fulfill the stated objectives, a device is designed for detecting cellular mechanical force. The device includes an array of micropillars located on the base, the micropillars are deformable in respond to a cellular mechanical force and a light-reflective layer is located on a top or an upper portion of the micropillars.

Further, in the device for detecting cellular mechanical force, the base is light transmissive; the top of the micropillars has the light-reflective layer, and bodies of the micropillars are capable of transmitting light.

Furthermore, in the device for detecting cellular mechanical force, a surfaces of the micropillars includes an anti-reflection layer.

Additionally, in the device for detecting cellular mechanical force, the light-reflective layer is composed of a combination of one or more of the following: metal foillayer, metal oxide or metal salts, ultrafine glass beads or micro-prisms, and organic reflective materials, or a combination thereof.

Moreover, in the device for detecting cellular mechanical force, all or some of the micropillars have a cell adhesion substance coated at a top surface thereof.

Furthermore, in the device for detecting cellular mechanical force, the substances with cell adhesion properties include one or more of the following substances: extracellular matrix molecules, including collagen, fibronectin, laminin, vitronectin, or elastin; simulated extracellular matrix substances, including peptides containing RGD adhesion sequences; substances with mechanisms to promote cell adhesion, including polylysine; and substances interacting with cell surface receptors.

Additionally, in the device for detecting cellular mechanical force, a portion of the top end surfaces of some micropillars in a preset area of the array of micropillars are provided with substances that have cell adhesion properties.

Moreover, in the device for detecting cellular mechanical force, micropillars on the top end surface without substances that have cell adhesion properties are provided with substances that have cell adhesion inhibition properties.

Furthermore, in the device for detecting cellular mechanical force, the cross-sectional shape of the micropillars is circular, elliptical, or polygonal.

Additionally, in the device for detecting cellular mechanical force, dimensions of the micropillars and the array include: heights of the micropillars in a range of 10nm to 500µm, inter-pillar in a range of 10nm to 50µm, and the top surface of the micropillars in a range of 50nm to 50µm.

Further, the device for detecting cellular mechanical force also includes cell restraining mechanisms comprising one or more restraining surfaces, the one or more restraining surfaces are flat surface or curved surface, and are perpendicular to the base, wherein the restraining surface has a height greater than the micropillar and enclosures a preset number of the micropillars.

The inventors also provide a system for detecting cellular mechanical force, comprising the device for detecting cellular mechanical force, the light signal generation device, and the light signal detection device as described in the above technical solutions.

The light signal generation device having a light source, and the light emitted by the light source being illuminated to the light-reflective layer of the micropillars through an incident light path.

The light signal detection device configured for detecting light reflected from the light-reflective layer of the micropillars, and the light reflected by the light-reflective layer enters the light signal detection device through a reflection light path.

Further, in the system for detecting cellular mechanical force, the base of the device for detecting cellular mechanical force is light transmissive, and the bodies of the micropillars are capable of transmitting light; the top of the micropillars have the light-reflective layers.

The light emitted by the light source is illuminated from the base to the light-reflective layer of the micropillars through the incident light path.

The light signal detection device is configured for detecting the light reflected from the light-reflective layer.

Further, a system for detecting cellular mechanical force also includes a light signal analysis device for analyzing the light signals.

The inventors also provide a method for detecting cellular mechanical force, comprising the following steps:

> First, emitting light using the light signal generation device in the detection system for cellular mechanical force as described above.
> Secondly, detecting the light after it has interacted with the detection device for cellular mechanical force using the light signal detection device in the detection system for cellular mechanical force as described above.

Further, the method for detecting cellular mechanical force, comprising using a light signal analysis device to compare and analyze the reflected light before interactions through the cell and device and after interactions through the cell and device to obtain cellular mechanical force information including a magnitude, direction, or frequency of the cellular mechanical forces.

The inventors also provide a method for detecting cellular states, comprising: obtaining cellular mechanical force information through a system for detecting cellular mechanical forces as described above, or through a method for detecting cellular mechanical forces as described above, and determining the cell status based on the cellular mechanical force information.

wherein the cell status includes cell adhesion, viability, differentiation/activation, proliferation, and/or migration.

Further, the cell status is either a static cellular state or a real-time cellular state.

The inventors also provide a method for cell identification, obtaining cellular mechanical force information through a system for detecting cellular mechanical forces as described above, or through a method for detecting cellular mechanical forces as described above, and distinguishing cell types based on the cellular mechanical force information.

Further, in the method for cell identification, the step of "utilizing any of the detection systems for cellular mechanical force or any of the methods for detecting cellular mechanical force as described above to obtain cellular mechanical force information, and distinguishing different types of cells based on the obtained cellular mechanical force information" specifically includes:

First, obtaining cell information, where the cell information includes the cellular mechanical force information of a point within the cell obtained by the detection device for cellular mechanical force, and the cellular mechanical force information includes the magnitude of the cellular mechanical force at that point.

Secondly, preprocessing the cell information to form structured cell information, where the structured cell information includes the number of cells, the number of cell features, and feature information for each cell feature.

Thirdly, using supervised, unsupervised, or semi-supervised machine learning to establish a cell feature model using structured cell information as input data, and applying the cell feature model to classify or cluster cells of unknown types or states.

Furthermore, in the method for cell identification, the cellular mechanical force information also includes the direction of the cellular mechanical force at that point.

Further, in the method for cell identification, the cellular mechanical force information also includes the variation of the magnitude or direction of the cellular mechanical force at that point over a certain time interval.

Further, in the method for cell identification, the cell information also includes cell morphology information.

The inventors also provide a method for preparing a cellular mechanical force detection device, comprising the following steps: forming a light-reflective layer on the top or upper half surface of the micropillars to obtain micropillars with light-reflective layers on the top or upper half surface.

Further, in the method for preparing the cellular mechanical force detection structure, before the step of forming a light-reflective layer on the top or upper half surface of the micropillars, further comprising:

Coating the entire micropillars with an anti-reflective layer uniformly;
Removing the anti-reflective layer from the top or upper half surfaces of the micropillars.

Further, in the method for preparing the cellular mechanical force detection structure, the step of "forming a light-reflective layer on the top or upper half surface of the micropillars to obtain micropillars with light-reflective layers on the top or upper half surface" includes: uniformly sputtering a layer of reflective metal on the top or upper half surface of the micropillars to obtain micropillars with metallic light-reflective layers on the top or upper half surface.

Further, in any of the above methods, the sample can be a single cell or any form of multicellular aggregate formed by two or more cells. The present application is not limited to various forms of single cells or multicellular aggregates formed by two or more cells.

Compared with the prior art, the present application brings the following beneficial effects:

First, high-throughput and low-cost: compared with the existing TFM and ordinary array of micropillars, the technical solution of the present application gets rid of the dependence on the microscope and greatly simplifies the operation process, because there is no need for high-resolution imaging through the microscope, only by monitoring the intensity of reflected light, high-throughput monitoring of cells can be achieved with low cost.

Secondly, single cell resolution: high resolution, real-time monitoring of each cell, can be combined with other single cell analysis techniques to measure the heterogeneity of cell response to drugs; real-time monitoring: no fluorescence, can avoid phototoxic effects of laser on cells , so it is suitable for long-term monitoring, and can be used to study the long-term response of cells to drugs; high sensitivity: through the reflected signal, the micropillar deformation signal is amplified to increase the sensitivity of deformation monitoring. The detection of bending deformation of micro- and nano-pillars generally relies on optical systems (such as microscopes) for detection, but the smaller the size of the micropillars is, the higher the precision and resolution of the optical system are to be needed. For example, a micropillar with a width of 2 microns and a height of 6 microns requires an objective lens with a power of 20 times or more and a conjugate focus system for effective observation. However, the present application utilizes the principle of specular reflection to detect the attenuation of reflected light, and actually amplifies the signal of the deformation of the micropillars. It has been verified by experiments that the same signal can be observed under a 5x objective lens. Combing with a special reading system, it can effectively detect the deformation of micropillars without relying on high-magnification optical objective lenses, thereby greatly reducing system costs and effectively improving throughput.

Furthermore: it can simulate the cellular microenvironment; it can simulate the components and morphologies of the extracellular matrix, and it can be suitable for more abundant technical demand scenarios.

Furthermore: cellular mechanical forces of multicellular aggregates, such as tumor spheroids and organoids, can be detected, so that it can be applied to drug screening, regenerative medicine, gene editing, precision medicine, organ development, and disease modeling scenarios that require characterization of multicellular aggregates.

BRIEF DESCRIPTION OF THE DRAWINGS

FIG. 1 is a schematic structural view of a device for detecting cellular mechanical force according to the first embodiment of the invention.

FIG.2 shows scanning electron microscope (SEM) top-view imageimages of the micropillars of the device for detecting cellular mechanical force in the first embodiment of the invention. wherein a) is a top view of the device, and b) is a side view of the device, is a schematic structural view of a system for detecting cellular mechanical force related to the ninth embodiment of the invention.

FIG.4 is a schematic structural view of a system for detecting cellular mechanical force related to the tenth embodiment of the invention.

FIG. 5 shows SEM images of micropillars (made of polydimethylsiloxane) with a reflective layer (gold) at the top; wherein FIG. 5,a is an SEM image of the micropillars, FIG. 5,b is an elemental characterization diagram of the top area of the micropillars , and FIG. 5,c is an elemental characterization diagram of the side area of the micropillars (excluding the top area).

FIG. 6 includes a) and b), wherein a) is a fluorescent imaging diagram of a preset pattern of micropillar groups with fibronectin at the top and cell adhesion.

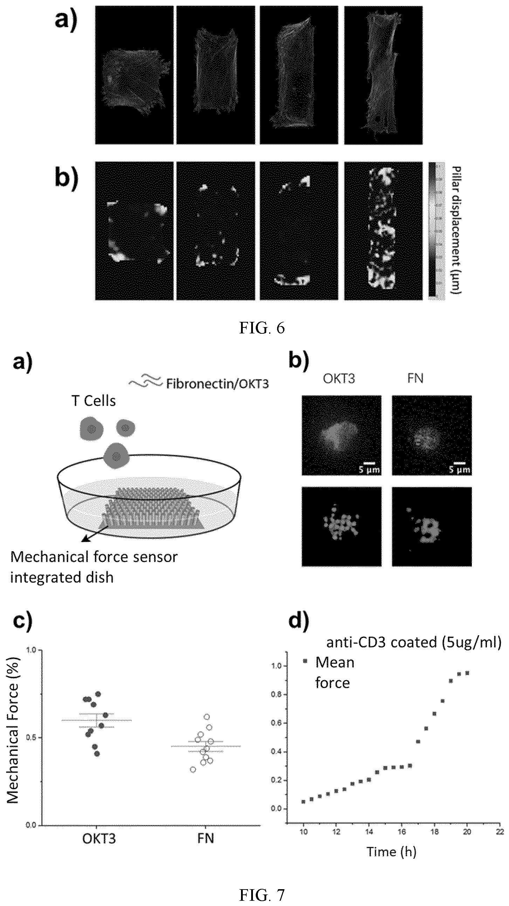

In FIG. 6, b) is a cell force distribution diagram calculated from the light reflection signal measured on t micropillar group with fibronectin and cell adhesion.

FIG. 7 includes a), b), c) and d), wherein a) illustrates an experimental diagram using OKT3 antibody as a substance with cell adhesion properties.

In FIG. 7, the upper part of b) shows fluorescent imaging diagrams of cells adhering to the top ends of micropillars coated with OKT3 antibody and fibronectin, respectively, while the lower part of b) displays a distribution diagram of cellular mechanical force magnitude calculated from the light reflection signal measured on the micropillars.

In FIG. 7, c) shows a comparison diagram of the mechanical magnitudes measured on surfaces coated with OKT3 antibody and fibronectin.

In FIG. 7, d) shows a dynamic change diagram of cellular mechanics after T cells are seeded on surfaces (top of micropillars) with OKT3 antibody.

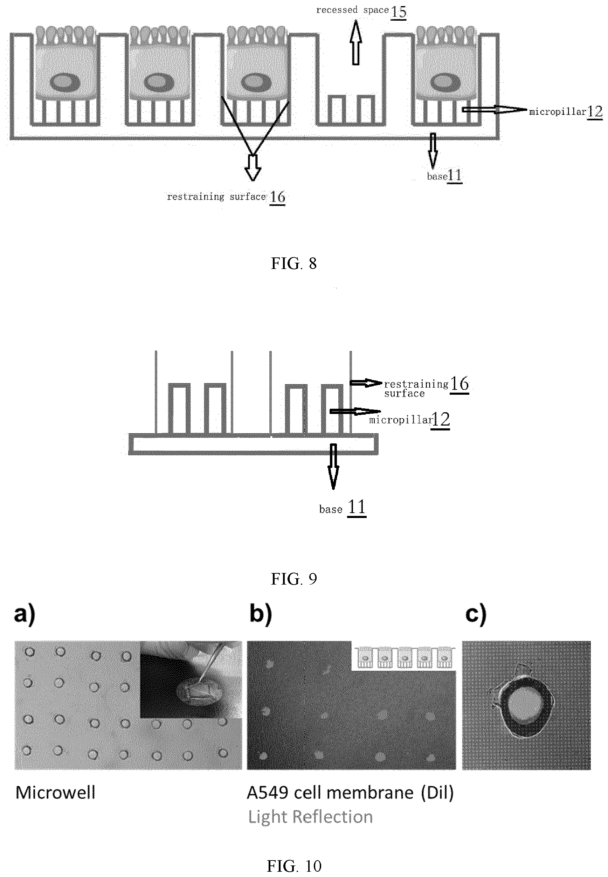

FIG. 8 is a schematic structural view a of a device for detecting cellular mechanical force with a cell restraining mechanism.

FIG. 9 is a schematic structural view b of a device for detecting cellular mechanical force with a cell restraining mechanism.

FIG. 10 includes a), b) and c), wherein a) shows a real image of a device for detecting cellular mechanical force using a silicon film as a cell restraining mechanism.

In FIG. 10, b) shows a fluorescence microscope image under light-reflective layer of a device for detecting cellular mechanical force using a silicon film as a cell restraining mechanism.

In FIG. 10, c) is an enlargement image of FIG. 10, b).

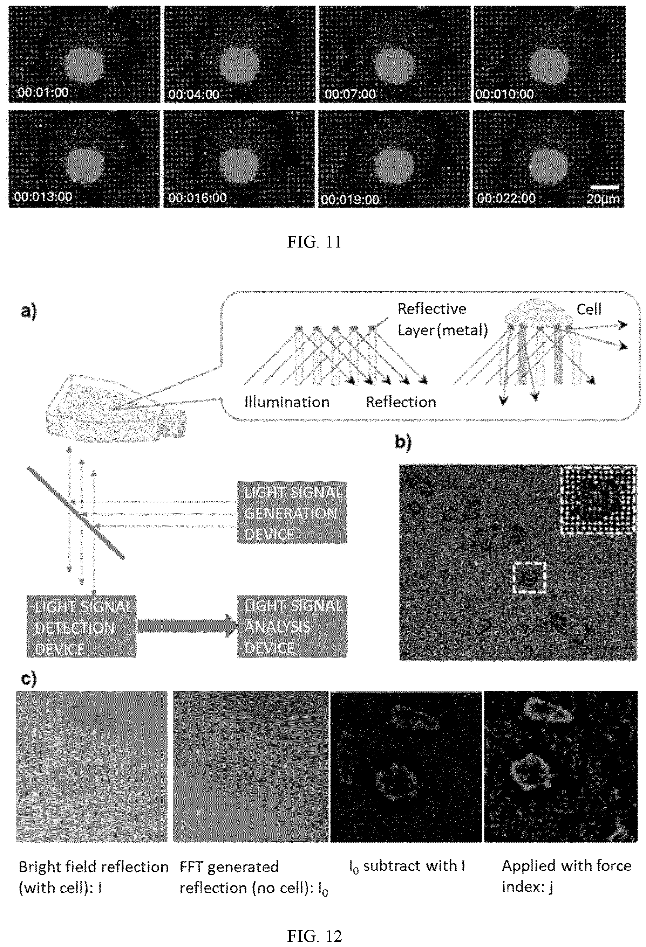

FIG. 11 shows a fluorescence microscope image of cellular mechanical forces monitored by the detection system according to the eleventh embodiment.

FIG. 12 includes a), b), c), wherein a) is a schematic structural view of the system for detecting cellular mechanical force according to the twelfth embodiment.

In FIG. 12, b) shows an image of the light-reflective signal obtained by the light signal detection device of the device for detecting cellular mechanical force.

In FIG. 12, c) shows a visualization effect diagram of the mechanical magnitude and distribution processed by the light signal analysis device according to the twelfth embodiment.

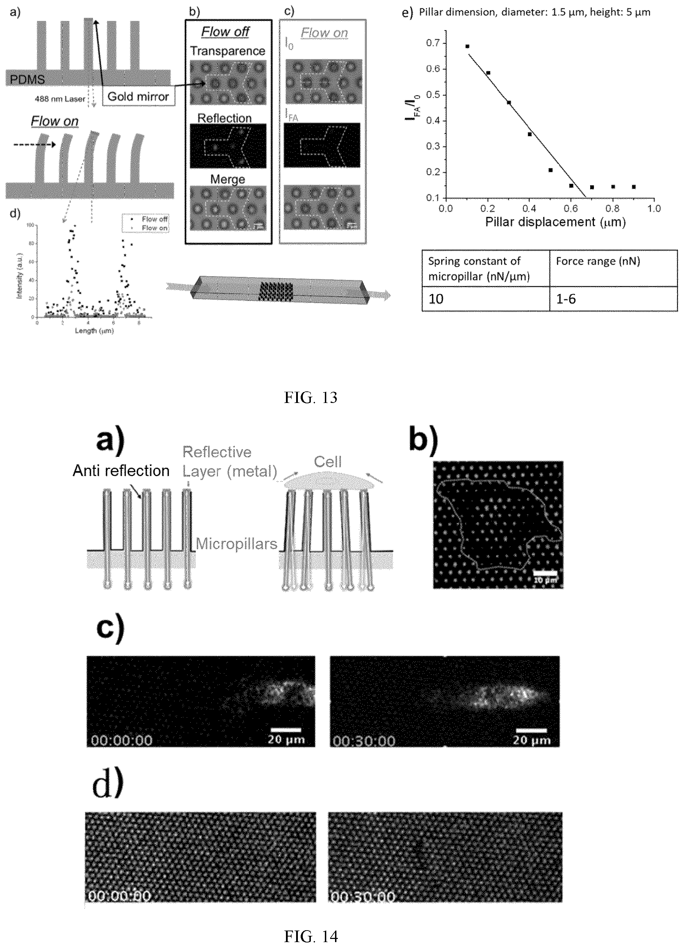

FIG. 13 includes a), b), c), d), e), wherein a) is a schematic structural view of a device for detecting cellular mechanical force in a microfluidic environment before and after flow on.

In FIG. 13, b) is a comparative diagram of bright field microscope images of micropillars, reflection light signal distribution diagrams, and their overlay effects before flow on; wherein the overlay effect diagrams refer to the combined effect diagrams created by overlaying the bright field microscope images and reflection light signal distribution diagrams of the micropillars.

In FIG. 13, c) is a comparative diagrams of bright field microscope images of micropillars, reflection light signal distribution diagrams, and their overlay effects after flow on, wherein the overlay effect diagrams refer to the combined effect diagrams created by overlaying the bright field microscope images and reflection light signal distribution diagrams of the micropillars.

In FIG. 13, d) shows the intensity values of the light-reflective signal before and after flow on.

In FIG. 13, e) shows the linear interval between light signal attenuation and top displacement of the micropillars.

FIG. 14 includes a), b), c) and d), wherein a) is a schematic structural view before and after the micropillars of a device for detecting cellular mechanical force come into contact with cells.

In FIG. 14, b) is a distribution diagram of the reflected light signal obtained by the light signal detection device.

In FIG. 14, c) is a monitoring diagram of cell migration process.

In FIG. 14, d) is a distribution diagram of the reflected light signal during cell migration process.

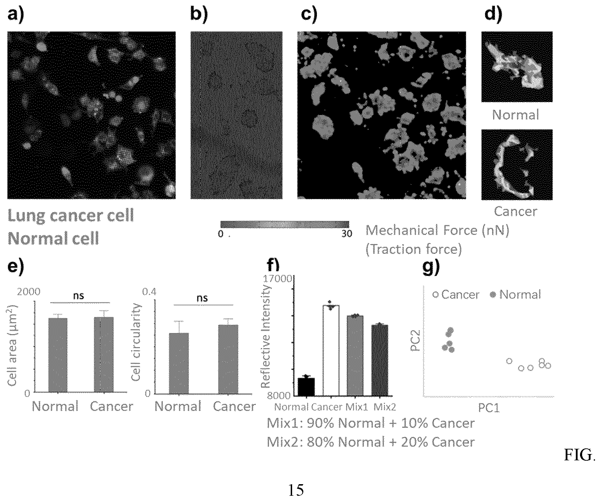

FIG. 15 includes a), b), c), d), e), f) and g), where a) is a fluorescence imaging diagram of a mixed system of healthy cells and lung non-small cell carcinoma cells.

In FIG. 15, b) is a distribution diagram of the light-reflective signal obtained by the light signal detection device of a device for detecting the cellular mechanical force.

In FIG. 15, c) is a visualization effect diagram of mechanical magnitude and distribution processed by the light signal analysis device.

In FIG. 15, d) is an enlargement diagram of representative single-cell cellular mechanical force distribution of healthy cells and lung non-small cell carcinoma cells from FIG. 15c.

In FIG. 15, e) is a comparative diagram of cell morphologies of healthy cells and lung non-small cell carcinoma cells.

In FIG. 15, f)is a comparative diagram of reflection signal intensity of healthy cells, lung non-small cell carcinoma cells, and a mixture of these two types of cells at different ratios.

In FIG. 15, g) is a cluster analysis diagram based on structured cell information obtained from FIG. 15c.

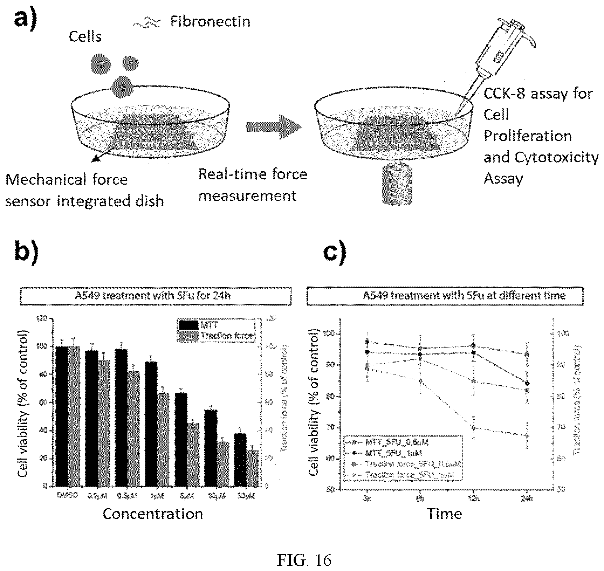

FIG. 16 includes a), b), and c), wherein a) is a schematic diagram of operational procedure for a cell viability detection method.

In FIG. 16, b) is a comparison diagram of cell viability measured by the MTT assay and cell viability reflected by cellular mechanical forces of A549 cells treated with different doses of 5FU for 24 hours.

In FIG. 16, c) is a comparison diagram of cell viability measured by the MTT assay and cell viability reflected by cellular mechanical forces of A549 cells treated with different doses of 5FU for different time.

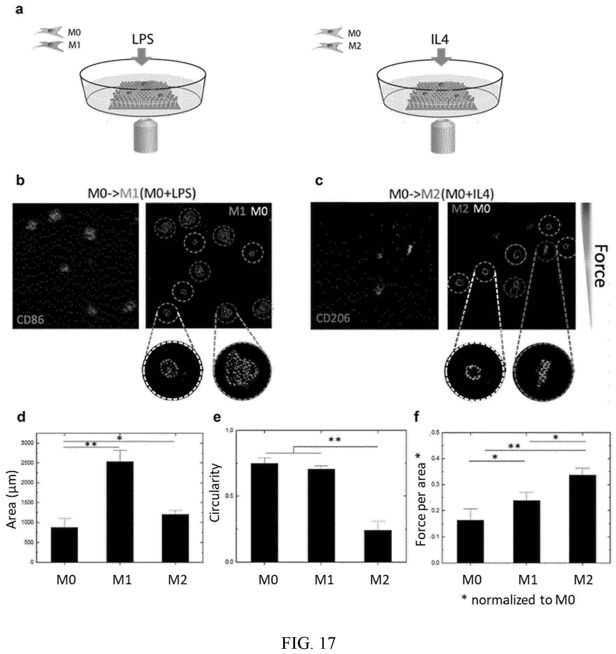

FIG. 17 includes a), b), c), d), e) and f), wherein a) is an operation process diagram for a cell state detection method.

In FIG. 17, b) is a fluorescence microscope image of M0 macrophages differentiating into the M1 state.

In FIG. 17, c) is a fluorescence microscope image of M0 macrophages differentiating into the M2 state.

In FIG. 17, d) is a comparison diagram of cell adhesion areas for M0 macrophages, and cells in the M1 and M2 states.

In FIG. 17, e) isa comparison diagram of cell roundness for M0 macrophages, and cells in the M1 and M2 states.

In FIG. 17, f) isa comparison diagram of cellular mechanical forces for M0 macrophages, and cells in the M1 and M2 states.

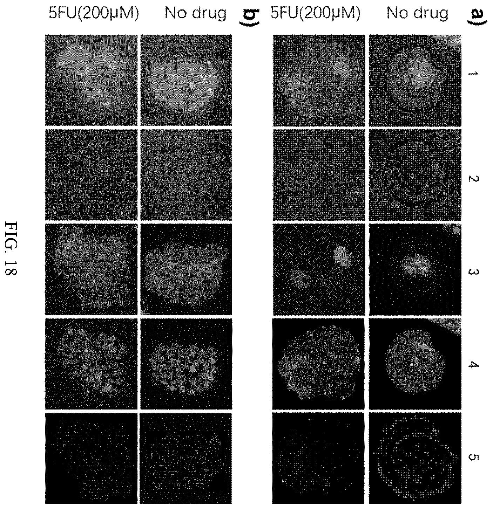

FIG. 18 includes a) and b), wherein a) represents characterization images of tumor cell aggregates with a first morphology before and after action of 5-FU; from left to right, the images show a mixed image of cell membrane fluorescence and reflection signals (1), light-reflective signal (2), cell nucleus (3), cell membrane (4), and a cell force visualization image processed by the light signal analysis device (ImageJ) (5), separately.

In FIG. 18, b) represents characterization images of tumor cell aggregates with a second morphology before and after action of 5-FU; from left to right, the images show a mixed image of cell membrane fluorescence and reflection signals (1), light-reflective signal (2), cell nucleus (3), cell membrane (4), and a cell force visualization image processed by the light signal analysis device (ImageJ) (5), separately.

Labels in the drawings: 1, device for detecting cellular mechanical force; 2, light signal generation device; 3, light signal detection device; 4, light signal analysis device; 5, beam splitter; 11, base; 12, micropillar; 13, light-reflective layer; 15, recessed space; 16, restraining surface.

DETAILED DESCRIPTION OF PREFERRED EMBODIMENTS

To provide a detailed description of the technical content, constructional features, objectives, and effects of the technical solutions, the following explanations are provided in conjunction with specific embodiments and accompanying drawings.

First Embodiment: A device for detecting cellular mechanical force

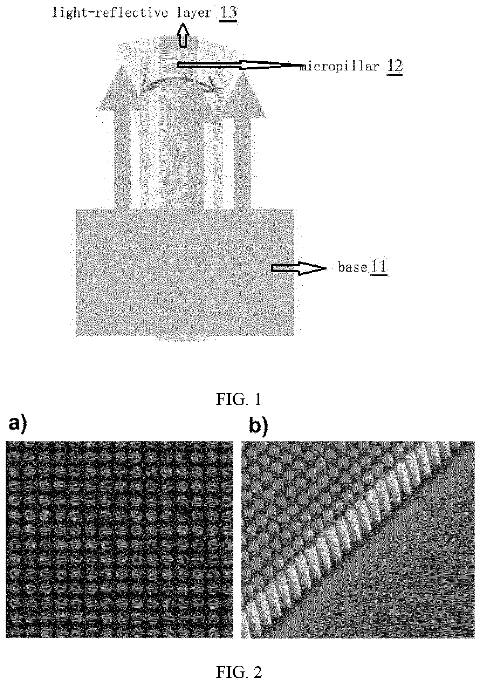

Referring to FIG. 1 for a schematic structural view of a device for detecting cellular mechanical force. The device illustrated includes a base 11 and multiple micropillars 12 positioned on the base, which deform in response to cellular mechanical forces. The base 11 is light transmissive, and the tops of the micropillars are coated with a light-reflective layer 13, with a thickness of 5nm (in other embodiments, the thickness of the light-reflective layer 13 can range from 5nm to 20nm, depending on the coating thickness and the coating material. The choice of the coating thickness, given the same coating material, should ensure light transmission effectiveness, stability of the micropillar structure, and secure attachment to the micropillars). The micropillars 12 is capable of transmitting light, the clusters of arrows in opposite directions in the FIG. 1, represent incident rays and reflected rays.(Note: The term of coating in this embodiment signifies that the light-reflective layer 13 can be prepared by a coating process, but it is not limited to being prepared exclusively by such a process.).

Referring to FIG. 2 for scanning electron microscope (SEM) images of the actual micropillars 12 of the device in this embodiment. FIG. 2,a is a top view and FIG. 2,b is a side view. As shown in FIG. 2, the microstructure of the micropillars in the device is uniform and size-controllable, offering more precise mechanical measurements than existing devices for detecting cellular mechanical force.

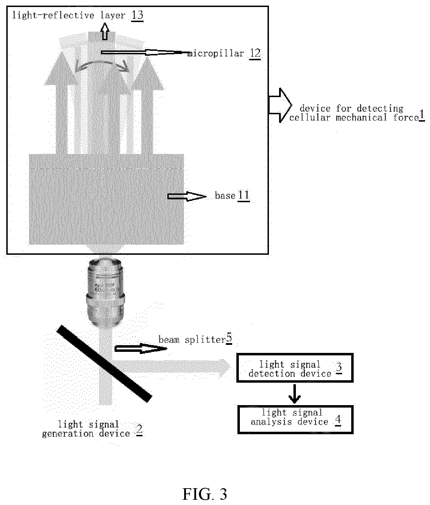

When the device for detecting cellular mechanical force 1 described in this embodiment is utilized, the number of micropillars 12 will exceed one. Referring to FIG. 3 for a schematic structural view of a system for detecting cellular mechanical force related to the ninth embodiment of this invention. FIG. 3 aids in understanding this embodiment. Besides the device for detecting cellular mechanical force] 1 described herein, the system shown in FIG. 3 also involves a light signal generation device 2 with light source positioned below the base 11 and a light signal detection device 3. The light emitted from the light source illuminates the light-reflective layer of the micropillars 12 through the base 11 characterized by its light transmissivity. The light signal detection device 3 is for detecting light reflected from the light-reflective layer 13 at the top of the micropillars. After passing through the reflective light path and interacting with beam splitter 5, the processed reflected light enters the light signal detection device 3. After obtaining the reflected light signal, a light signal analysis device 4 can compare and analyze the reflected light before and after the interaction of the device 1 for detecting cellular mechanical force with the cells to be tested, thereby obtaining cellular mechanical force information. Without force, the micropillar should remain upright, maximizing light reflection layer; when the micropillars come into contact with cells, they bend under the action of cellular mechanical forces, causing a reduction in the level of reflected light. Thus, greater cellular mechanical forces correspond to lower light-reflective signals, allowing the magnitude of cellular mechanical forces at that point to be easily inferred by observing the intensity of the light-reflective signals.

Moreover, the measurement light source in this embodiment may utilize infrared laser of certain intensity. Traditional methods requiring high-resolution imaging might introduce phototoxicity to cells or photoquenching of samples when using lasers. However, in present application, if only measuring reflected signals, the impact of infrared lasers of certain intensities on cells can be negligible, making it suitable for long-term monitoring of cells.

Second Embodiment: A Device for Detecting Cellular Mechanical Forces

Differing from the first embodiment, the micropillars 12 not only have a light-reflective layer 13 on their top ends but also on the upper half of their periphery surfaces (i.e., the surface connecting the two end surfaces of the pillar body). In practice, other embodiments avoid applying the light-reflective layer 13 to the lower half of the periphery surfaces due to inferior results; as long as the light-reflective layer 13 is applied to the upper half of the periphery, the intended detection effect of the invention can be achieved. That is, in some embodiments, the light-reflective layer 13 could even be applied to any local area on the upper side or top end without covering the entire upper half or the entire top end, still achieving the intended purpose, though there may be differences in the data obtained and the results of subsequent calculations.

Additionally, the definitions of periphery surface and end surface are introduced in the first and second embodiments, meaning that a standalone pillar typically has two end surfaces connected by a periphery surface. However, in the invention, due to the presence of the base, the micropillar has only one end surface, the top end, with the other end fixedly connected to or integrally formed with the base. In other embodiments, the top end surface may seamlessly connect to the periphery surface without a distinct boundary or intersection line as shown in the first or second embodiment. In such cases, the placement of the light-reflective layer 13 is also considered to be on the upper half part of the pillar body, not strictly limited to the end surface or periphery surface.

Third Embodiment: A Device for Detecting Cellular Mechanical Force

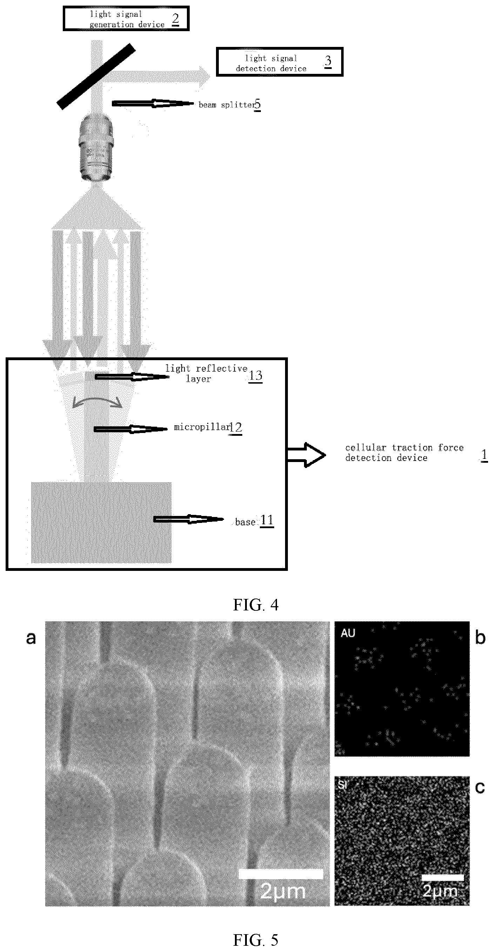

Referring to FIG. 4, which is a schematic structural view of a system for detecting cellular mechanical force related to the tenth embodiment of the invention, illustrating the device 1 for detecting cellular mechanical force in this embodiment. Differing from the first and second embodiments, there is no requirement for the transmissivity of the base 11 and the bodies of the micropillars 12 of the array of micropillars. They can be opaque, transmissive, or semi-transmissive. In such cases, only the positions of the light signal generation device 2 and the light signal detection device 3 need to be adjusted to above the base 11. Whenever the micropillars bend, the light signal received by the light signal detection device 3 changes relative to that when the micropillars are upright and undeformed. Analyzing the change in light signals before and after bending can also yield the relative magnitude of cellular mechanical forces, which, after calibration against standard values, can provide absolute values of cellular mechanical forces.

Fourth Embodiment: A Device for Detecting Cellular Mechanical Force

Referring to FIG. 1, this embodiment differs from the first to third embodiments in that, on the surface of the micropillars 12, areas other than those with the light-reflective layer 13 are equipped with an anti-reflective layer . The design can reduce the interference from the light-reflective signals that may arise from the surface of the pillars, enhancing the signal-to-noise ratio and rendering the detection results more accurate.

In some embodiments, the light-reflective layer 13 can be a layer of gold foil. In other embodiments, the light-reflective layer 13 may also be other types of metallic layers or reflective materials with light-reflecting capabilities. The choice of material, due to differences in reflective effects, ease of preparation of the light-reflective layer, and cost, may vary and should be considered and selected based on specific conditions.

Across the first to fourth embodiments, the cross-sectional shape of the micropillars 12 is circular. In other embodiments, the cross-sectional shape of the micropillars 12 could also be elliptical or polygonal. The variation in the cross-sectional shapes in different embodiments of this invention can achieve different objectives. For example, a circular cross-section has isotropic characteristics, meaning the mechanical properties of the micropillars themselves are not sensitive to direction. In contrast, an elliptical cross-section exhibits anisotropy, meaning the micropillars' mechanical properties are sensitive to direction. This sensitivity allows for control over the sensitivity to force fields in different directions and can somewhat regulate cell orientation (most cells exhibit asymmetric geometric shapes. The term of orientation in this invention refers to the asymmetry, polarity, or directionality exhibited by cells. For instance, if an ellipse approximates the shape of a cell's projection, the major axis of the ellipse could represent the cell's orientation). Because an elliptical cross-section has a major axis and a minor axis, pushing the micropillar along the minor axis is much easier than along the major axis, thereby resulting in greater deformation under relative force conditions. In some extended embodiments, seeding cells on such micropillars, where there is an anisotropic mechanical interactions among the cells and the micropillars, could lead to cell growth along a particular side. When applied in fluids, it can be used to determine the direction of fluid flow.

In the first to fourth embodiments, the dimensions of the array of micropillars are: pillar height 10nm to 500$\mu$m, inter-pillar distance 10nm to 50$\mu$m, and the diameter of the pillar's top surface 50nm to 50$\mu$m. Micropillars within this size range can meet the basic requirements for sensors, i.e., they are at least deformable and not prone to collapse. Additionally, manipulating the dimensions of the array of micropillars can achieve functionalities such as controlling the deformation capabilities of the pillars by adjusting the aspect ratio (the ratio of height to cross-sectional diameter/edge length/major diameter at the level of the pillars) to better simulate the in vivo environment of organ tissues (e.g., bone tissues of different hardness and neural tissues).

Moreover, the overall specifications of the array, or the number of micropillars 12 on a certain area of the basell, will affect the ligand density, i.e., the number of adhesion points that cells can find on the surface. A sparser array of micropillars 12 means fewer adhesion points for the cells, significantly impacting the cell behavior.

The cross-sectional area of the pillars also influences the cell adhesion behavior, because the formation of focal adhesions requires a certain area. If the pillars are nano-sized, the small cross-sectional area of the pillars can affect the formation of focal adhesions.

In summary, combining the material characteristics with certain micro-/nano array dimensions, it can achieve more desirable effects in terms of cell support, chip stability, and measurement accuracy. Adjusting the distribution of the array of micropillars can also regulate and affect the cell adhesion states to some extent.

In the first to fourth embodiments, the material of the micropillars 12 is polydimethylsiloxane (PDMS). In other principal embodiments of this invention, the material of the micropillars 12 could also be other polymer materials, such as silicone-based polymers, photoresist polymers, conductive polymers, and thermosensitive polymers. The main reason for using polymer materials in the principal embodiments of this invention is that polymer materials currently have suitable deformability for the applications of this invention. However, the implementation of this invention does not limit the material of the pillars to polymers; it can and should be extended to all materials with the corresponding deformability, capable of realizing the inventive concept of this invention. In short, the material of the pillars must satisfy certain conditions of deformability and, in some embodiments, a certain degree of transmissivity, although the latter is not a necessary condition for all embodiments. When using materials with limited transmissivity to fabricate the pillars, the inventive concept of this invention can still be realized by appropriately positioning the light signal generation and detection devices.

Overall, the hardness (deformability) of the micropillars 12 can be adjusted based on actual needs through multiple technical dimensions such as size (primarily Aspect Ratio), choice of material type, control over the degree of cross-linking of polymer materials, and chemical or physical surface treatments.

Referring to FIG.5, which shows scanning electron microscopy images of a micropillar (polydimethylsiloxane) with a reflective layer (gold) at the top position; wherein, FIG. 5,a shows the scanning electron microscopy image of the pillar; FIG. 5,b shows the elemental characterization of the top area of the pillar; FIG. 5,c shows the elemental characterization of the side area of the pillar (excluding the top area). The scanning electron microscopy images in FIG. 5 characterize the composition of the micropillar, confirming the presence of Au elements at the top of the pillar and Si elements at other positions of the pillar.

Fifth Embodiment: A Device for Detecting cellular mechanical force

This embodiment differs from the first to fourth embodiments in that some of the micropillars 12 in the array of micropillars have their top surfaces coated with cell adhesive substances . In this embodiment, collagen, a type of extracellular matrix molecule, is used. In other embodiments, combinations of one or several types of extracellular matrix molecules, including collagen, fibronectin, vitronectin, laminin, and elastin, could be used. In other embodiments, the top surfaces of all or some of in the array of micropillars could be coated with other types of cell adhesive substances , such as analogs of the extracellular matrix containing RGD adhesion sequences, substances that promote cell adhesion, including polylysine, or materials that interact with cell surface receptors.

The top surfaces of micropillars 12 have such cell adhesive substances can effectively promote cell attachment to the micropillars 12, thereby regulating cell attachment, proliferation, migration, state, differentiation, and other behaviors. Furthermore, if cell adhesive substances, such as fibronectin and other extracellular matrix proteins, are applied to the top surfaces of some micropillars in a preset area of the array, these micropillars can form certain shapes. Thus, cells tend to adhere to the micro pillars in specific locations and shapes, allowing for high-throughput mechanical measurements through controlling cell size, shape, and orientation characteristics.

Sixth Embodiment: A Device for Detecting Cellular Mechanical Force

This embodiment differs from the fifth embodiment in that, as described in the fifth embodiment, some of the micropillars 12 in the array have their top surfaces coated with cell adhesive substance. In the embodiment, the surfaces (either the top or side surfaces) of those micro pillars 12 not coated with cell adhesive substances are coated with cell adhesion inhibiting substance such as F-127. The cells to preferentially adhere to micro-pillars at specific locations and shapes, thus allowing for high-throughput mechanical measurements while controlling the size, shape, and orientation characteristics of the cells.

Seventh Embodiment: A Device for Detecting Cellular Mechanical Force

This embodiment differs from the first through fourth embodiments in that the top surfaces of the array of micropillars are patterned with substances that promote cell adhesion to form preset patterns. Specifically, cell adhesion molecules can be printed in specific patterns through microprinting technologies to promote cell attachment in these areas. The preset patterns can include shapes such as triangles, quadrilaterals, polygons, circles, ellipses, etc. The purposes of these preset patterns include: firstly, controlling cell-to-cell contact through patterns formed by cell adhesive substances to facilitate the acquisition of high-throughput data. Secondly, standardizing cell shapes to achieve dimensionality reduction in data processing, thereby simplifying analysis. Thirdly, by restricting cell attachment areas, it is possible to control cell size, shape, orientation, differentiation state, and even regulate the cell mechanical state through actin filament control, meeting certain specific technical requirements.

In another embodiment similar to this one, the parts of those array of micropillars not printed with the preset pattern can be coated with substances that inhibit cell adhesion, such as BSA (Bovine Serum Albumin) or F127 (a non-ionic polymeric surfactant), to inhibit cell attachment in these areas, thus achieving directed attachment, control of cell morphology, or simulation of specific cell microenvironments.

In other embodiments, fibronectin (FN) is used as an illustrative example of a cell adhesive substance, but this is not intended to limit the scope of the invention. Using polydimethylsiloxane microstamps with protruding square and rectangular patterns coated with fibronectin, the fibronectin on the protruding parts of the stamp is transferred to the metal reflective layer at the top the micropillars through microcontact printing. Subsequently, the micropillars are immersed in an F-127 solution, rendering the parts not coated with fibronectin inhibitory to cell adhesion. After thoroughly washing the micropillars with saline, fibroblasts stained with cell membrane dye are seeded on the surface of the micropillars, followed by fluorescence imaging of the cells (as shown in FIG. 6,a) and high-resolution measurement of the intra cellular mechanical force field (as shown in FIG. 6,b). Referring to FIG.s 6a and 6b, FIG. 6,a shows the fluorescence imaging of cells adhering to the preset pattern formed by micropillars coated with fibronectin, demonstrating that cell adhesion is restricted to the areas with fibronectin; based on this, mechanical monitoring of cells can be conducted by restricting cell attachment areas, thereby controlling cell size, shape, orientation, differentiation state, etc.; and FIG. 6,b shows the distribution of cellular mechanical forces derived from the light-reflective signals measured on the micro-pillars.

In other embodiments, the OKT3 antibody (a substance that interacts with cell surface receptors) or fibronectin (FN) is used as an illustrative example of a cell adhesive substance, but this is not intended to limit the scope of the invention. Refer to FIG.s 7,a-d, FIG. 7, a shows a schematic diagram of an experiment using the OKT3 antibody as the cell adhesive substance; the upper part of FIG. 7b shows fluorescence images of cells adhering to the tops of micropillars coated with OKT3 antibody or fibronectin; the lower part of FIG. 7b shows the distribution of light-reflective signals (reflecting the magnitude of cellular mechanical forces) measured on the micropillars; FIG. 7c shows a comparison of mechanical magnitudes measured on surfaces coated with OKT3 antibody or fibronectin; and FIG. 7d shows the dynamic changes in cell mechanics occurring after T cells are seeded on the surface (top of the micropillars) coated with OKT3 antibody. Specifically, in the aforementioned experiments, some micropillars' tops within the same or different device for Detecting cellular mechanical force can be coated with OKT3 antibody or fibronectin, and T cells are seeded on the surfaces of the device for detecting cellular mechanical force coated with cell adhesive substances. As shown in FIG.s 7,a-d, a device for detecting cellular mechanical force coated with substances that interact with cell surface receptors (such as the OKT3 antibody) or fibronectin (FN) on the surface of micropillars can be used for real-time monitoring of the cellular mechanical force impact and interactions of these substances on cells.

Eighth Embodiment: A Device for Detecting cellular mechanical force

This embodiment differs from the first through seventh embodiments in that the device for detecting cellular mechanical force further includes a cell restraining mechanism. This cell restraining mechanism includes one or several restraining surfaces 16, which are perpendicular to the plane where the base 11 is located, and connected to or integrally formed with the base 11, and the height of the restraining surfaces 16 exceeds that of the micropillars 12, enclosing a preset number of micropillars 12 therein.

The purpose of the cell restraining mechanism set in this embodiment is for single cell isolation testing, i.e., to avoid contact or adhesion between cells during testing and to restrict cell morphology, thus facilitating high-throughput testing. Depending on different requirements, the number of restraining surfaces 16 in the cell position restraining mechanism or the shape formed can be different. For example, the restraining surfaces 16 included in the cell position restraining mechanism could be a cylindrical surface, or three planes forming a triangular cross-sectional shape enclosing a certain number of micropillars by end-to-end connection, or four planes perpendicular to each other forming a rectangular shape enclosing a certain number of micro-pillars by end-to-end connection, or N planes enclosing an N-sided shape by end-to-end connection, or even a curved surface with a quasi-circular cross-section. In other words, the cross-sectional shape formed by restraining surfaces 16 is a controllable closed shape, and the area thereof (or understood as the number of micropillars that can be accommodated in the space thereof) is also controllable.

In practical implementations, depending on the preparing process, the cell restraining mechanism can also show the following forms:

A. Referring to FIG. 8, which is a schematic structural view of a device for detecting cellular mechanical force with a cell restraining mechanism. In this diagram, the cell restraining mechanism is integrally formed with the base 11, i.e., the material forming the cell restraining mechanism has several recessed spaces 15 , whose walls act as restraining surfaces 16, the depth of recessed spaces 15 is the height of restraining surfaces 16, the bottom of recessed spaces 15 serves as base 11, and each recessed space 15 contains several micropillars 12.

Referring to FIG. 9, which is a schematic structural view of a device for detecting cellular mechanical force with a cell-restraining mechanism. In this diagram, the restraining surfaces 16 are structures bonded onto the base 11.

Ninth Embodiment: A Device for Detecting Cellular Mechanical Force

This embodiment differs from the eighth embodiment in that, in this embodiment, the cell-restraining mechanism is made of a silicon film.

Specifically, refer to FIG.s 10a-10c, FIG. 10,a is a real image of a device for detecting cellular mechanical force using a silicon film as the cell-restraining mechanism. In FIG. 10,a, after laser perforation, the silicon film is adhered to the base, with each hole containing a micropillar, using the silicon film to restrict cell morphology and migration while controlling cell-to-cell contact or adhesion. FIG. 10,b is a fluorescence microscopy image under light reflection using a silicon film as the cell-restraining mechanism. FIG. 10,c is an enlarged image of FIG. 10,b. In some embodiments, each hole in the silicon film can be sized to fit a single cell, suitable for single-cell adhesion, thus restricting cell contact, morphology, and migration range.

Tenth Embodiment: A System for Detecting Cellular Mechanical Force

A system for Detecting cellular mechanical force includes the device 1 for detecting cellular mechanical force as described in either the first or second embodiment, a light signal generation device 2, and a light signal detection device 3. Both the light signal generation device 2 and the light signal detection device 3 are located beneath the base 11 of the device 1 for detecting cellular mechanical force. The light signal generation device 2 includes a light source, which emits light, and the light can be illuminated to the light-reflective layer 13 of the micropillars 12 through an incident light path (successively passing through the transmissive base and the transmissive micropillar bodies), with reflection occurring. The reflected light then travels through the reflective light path (successively through the transmissive micropillar bodies and the transmissive base) into the light signal detection device 3. The light signal detection device 3 is capable of capturing the light-reflective signals before and after contact between the micropillars 12 and the cells. In other embodiments, this system for Detecting cellular mechanical force also includes a light signal analysis device 4, which can obtain cellular mechanical force information, including the magnitude, direction, and changes over a certain time range of cellular mechanical forces, by comparing, analyzing, and calculating the light-reflective signals before and after contact between the micropillars 12 and the cells.

Eleventh Embodiment: A System for Detecting Cellular Mechanical Force

Referring to FIG. 4, which illustrates a system for Detecting cellular mechanical force related to the eleventh embodiment of this invention. This system includes the device 1 for detecting cellular mechanical force as described in the third embodiment, a light signal generation device 2 and a light signal detection device 3. Both the light signal generation device 2 and the light signal detection device 3 are located above the base 11 of the device 1 for detecting cellular mechanical force. The light signal generation device 2 includes a light source, which emits light, and the light cancan be illuminated to the light-reflective layer 13 through an incident light path, with reflection occurring. The light signal detection device 3 is capable of capturing the light-reflective signals before and after contact between the micropillars 12 and the cells.

In embodiments of this invention, the light signal detection device can be a microscope, charge-coupled device (CCD), complementary metal-oxide-semiconductor (CMOS) sensor, photomultiplier tube (PMT), phototransistor (PT), film, or other light signal detection elements with similar functions, without specific restriction. It's noteworthy that in some embodiments, when using a microscope as the light signal detection device, there is no need to set up an independent light signal generation device. The device for detecting cellular mechanical force of this invention can be placed on the stage of the microscope, using the microscope's light source as the light signal generation device and the microscope's objective lens (a 5x objective lens is sufficient, without reliance on high-magnification optical lenses) as the light signal detection device. When using other light signal detection devices, such as a CCD, an independent light signal generation device is required. In embodiments of this invention, the light signal generation device can be an LED, halogen lamp, laser (e.g., infrared laser), or other light sources, without specific restriction.

The following details a specific introduction of the visualization process for monitoring cellular mechanical forces using a system related to this embodiment.

Referring to FIG. 11 for fluorescence microscopy images obtained using the system for detecting cellular mechanical force of this embodiment for monitoring cellular mechanical forces. Specifically, cells (e.g., fibroblasts in this embodiment) are placed on the micropillars of the device for detecting cellular mechanical force, and the light signal detection device (e.g., a microscope in this embodiment) can transform the cellular mechanical force information into the optical signals to form images for visualization and provide a real-time feedback on changes in cellular mechanical forces.

Twelfth Embodiment: A System for Detecting Cellular Mechanical Force

Referring to FIG. 4, which illustrates a system for Detecting Cellular Mechanical Force related to the twelfth embodiment of this invention. This system includes the device 1 for detecting cellular mechanical force as described in the third embodiment, a light signal generation device 2, a light signal detection device 3, and a light signal analysis device 4. Both the light signal generation device 2 and the light signal detection device 3 are located above the base 11 of the device 1 for detecting cellular mechanical force. The light signal generation device 2 includes a light source, which emits light, and the light can be illuminated to the light-reflective layer 13 through an incident light path, with reflection occurring. A beam splitter 5, which can be a semi-transmissive and semi-reflective optical components or other equivalent optical components, aims to simplify the design of the optical path. The light signal detection device 3 is capable of capturing the light-reflective signals before and after contact between the micropillars 12 and the cells. The light signal analysis device 4 can obtain cellular mechanical force information, including the magnitude, direction, and changes over a certain time range of cellular mechanical forces, by comparing, analyzing, and calculating the light-reflective signals before and after contact between the micropillars 12 and the cells.

In embodiments of this invention, the light signal analysis device can be an optical image analysis software such as Imaged, Matlab, Fluoview, Python, or other optical image analysis components with similar functions, or a combination of these analysis software, without specific restriction.

The following details the detection and analysis process of a system for detecting cellular mechanical force related to this embodiment.

Referring to FIG.s 1-c, FIG. 12,Aa is a schematic structural view of the system for detecting cellular mechanical force structure; FIG. 12,b shows the image of the light-reflective signal obtained by the light signal detection device from the device for detecting cellular mechanical force; FIG. 12,c visualizes the magnitude and distribution of forces.

As shown in FIG. 12,a, each micropillar on the device for detecting cellular mechanical force is equipped with a metal reflective layer at the top and an anti-reflective layer on the sides. In the absence of cells, the light irradiating the micropillar from below is completely reflected and captured by the light signal detection device (e.g., a CCD camera). However, when cells adhere to the micropillars, the movement of cells generating cellular mechanical forces causes the pillars to tilt, thus reducing the reflection signal. After analyzing the light-reflective signal, the intensity of cellular mechanical forces can be calculated.

Furthermore, the images of the light-reflective signals from the device for detecting cellular mechanical force and in the enlarged images of local cell adhesion areas (as shown in FIG. 12,b) are collected by the light signal detection device (e.g., a CCD camera). Then, the light signal analysis device further processes the images from FIG. 12,b, converting these images into more intuitive mechanical magnitude and distribution visualization images as shown in FIG. 12,c.

The specific processing procedure is as follows: firstly, based on FIG. 12,b, a bright field reflection signal image (I, focused on the cell) is obtained; then, after filtering high-frequency signals through Fourier transform and performing an inverse Fourier transform, an image $I_0$ of the micropillar reflection signal under non-displacement conditions is calculated; subsequently, I and $I_0$ images are further processed, converting the reflection signal image into a more intuitive cell mechanics image (the $I_0$ signal value minus the I signal value) and standardizing to obtain a more intuitive visualization of cellular mechanical force intensity image j.

Thirteenth Embodiment: A Method for Calculating cellular mechanical forces, The Relationship Between Mechanics and light-reflective signals

This embodiment, in conjunction with any one of the system for detecting cellular mechanical force described in the tenth to twelfth embodiments or the cellular mechanical force detection method described in the fourteenth embodiment, illustrate the method for calculating cellular mechanical forces in the embodiments of this invention. It utilizes fluid as an applied force to validate the relationship between mechanics and light-reflective signals.

Referring to FIG. 13, wherein FIG. 13,a shows a schematic structural view of a device for detecting cellular mechanical force structure in a microfluidic environment before and after flow on; FIG.s 13,b and c compare the bright field microscope images of the micropillars and the light-reflective signal distribution before and after flow on, as well as their overlay effect images; FIG. 13,d presents the light-reflective signal intensity before and after flow on; FIG. 13,e depicts the linear relationship between light-reflective signal and micropillars displacement. The overlay effect image refers to the result of overlaying the bright field microscope image of the micro pillars with the light-reflective signal distribution image.

Initially, as shown in FIG. 13, the device for detecting cellular mechanical force is integrated into a microfluidic channel. When the external flow rate increases, the micropillars displace, and the angle of the surface reflective layer is changed (FIG.s 13,a to c). As evidenced by FIG. 13,d, the light-reflective signal weakens from strong before and after flow on. Specifically, by varying the flow rate to change the micropillar's displacement, and capturing the displacement of the micropillar's top relative to the base thereof using a confocal microscope, the cellular mechanical force (F) causing the micropillar to deflect by an angle δ can be calculated using the fol-

lowing formula:

$$F = k_{\text{bend}}\delta = \frac{3}{64}\pi E \frac{D^4}{L^3}\delta$$

Wherein F represents the cellular mechanical force causing the micropillar to deflect by an angle δ, E represents the Young's modulus, $k_{\text{bend}}$ represents the ideal spring constant of an isolated nano-pillar, D represents the diameter of the micropillar, and L represents the height of the micropillar.

Simultaneously, the light-reflective signal from the top of the micropillar is recorded. By plotting this signal against the micropillar displacement as in FIG. 13,e, a linear relationship graph between the light-reflective signal (reflecting cellular mechanical force) and micropillar displacement, as well as the linear range, can be obtained.

Fourteenth Embodiment: A Method for Detecting cellular mechanical force

A method for detecting cellular mechanical force, comprising the steps of:
Emitting light using the light signal generation device 2 in the system for detecting cellular mechanical force described in any one of the tenth to twelfth embodiments.

Detecting the light after interaction with the device 1 for detecting cellular mechanical force using the light signal detection device 3 in the system for detecting cellular mechanical force described in any one of the tenth to twelfth embodiments. The light signal detection device 3 is capable of capturing the light-reflective signals before and after contact between the micropillars 12 in the device 1 for detecting cellular mechanical force and the cells. In other embodiments, the light signal analysis device 4 in the system for detecting cellular mechanical force can obtain cellular mechanical force information, including the magnitude, direction, and changes over a certain time range of cellular mechanical forces, by comparing, analyzing, and calculating the light-reflective signals before and after contact between the micropillars 12 and the cells.

Refer to FIG.s 14a to 14d, FIG.s 14a and 14b show schematic structural views before and after the micropillars of a device for detecting cellular mechanical force come into contact with cells; FIG. 14,b shows the light-reflective signals captured by a light signal detection device (CCD sensor), with noticeable signal attenuation in areas around the cell where the force field is stronger; FIG. 14,c monitors the cell migration process (after staining the cell membrane, using a fluorescent light source for excitation and recording the migration process with the CCD sensor); FIG. 14,d shows the distribution of light-reflective signals during the cell migration process (recorded by the CCD sensor). FIG.s 14c and 14d reveal significant attenuation of the reflection signal in the areas where the cells exert force. FIG. 14,d demonstrates real-time monitoring of light-reflective signals during cell migration, and a real-time feedback on cellular mechanical forces during the migration process through monitoring the light-reflective signals by the light signal detection device, indicating that real-time feedback on the changes in cellular mechanical forces during cell migration can be achieved by monitoring the light-reflective signals.

Fifteenth Embodiment: A Method for preparing a Device for Detecting Cellular Mechanical Force

A method for preparing a device for detecting cellular mechanical force, comprising the steps of:
Applying a light-reflective layer 13 on the top or upper half periphery surface of the micropillars 12, obtaining micropillars 12 with a reflective layer on the top or upper half.

Sixteenth Embodiment: A Method for preparing a Device for Detecting Cellular Mechanical Force.

A method for preparing a device for detecting cellular mechanical force, including the steps of:

Uniformly coating the entire micropillars 12 with an anti-reflective layer;
Removing the anti-reflective layer from the top or upper half of the micropillars 12;
Applying a light-reflective layer 13 on the top or upper half of the micropillars 12.

Seventeenth Embodiment: A Method for preparing a Device for Detecting Cellular Mechanical Force.

This embodiment differs from the fifteenth and sixteenth embodiments in that the step of applying a light-reflective layer 13 on the top or upper half of the micropillars 12 specifically involves uniformly sputtering a layer of reflective metal on the top or upper half of periphery surface, obtaining micropillar with a metallic reflective layer on the top or upper half.

Eighteenth Embodiment: A Method for Cell Identification

This embodiment provides a method for cell identification, which involves using any of the system for detecting cellular mechanical force or the cellular mechanical force detection method described in the aforementioned embodiments to obtain cellular mechanical force information and distinguish different cell types based on this information.

In some embodiments, in the method for cell identification, the step of using the system for detecting cellular mechanical force described in any one of the above embodiments or the cellular mechanical force detection method described in any one of the above schemes to obtain the cellular mechanical force information, distinguishing different cell types for cell identification according to the cellular mechanical force information specifically includes:

S 1, Collecting cell information, the cell information includes the cellular mechanical force information of a specific point in the cell obtained by the detection device, the cellular mechanical force includes the magnitude of cellular mechanical forces at a specific point within the cells obtained from the device for detecting cellular mechanical force. Specifically, this involves using the light signal detection device (or in conjunction with the light signal analysis device) to collect cell information on multiple cells on the device for detecting cellular mechanical force, including collecting information on the magnitude of cellular mechanical forces at multiple points within each cell, thereby obtaining data on the magnitude of cellular mechanical forces at multiple points across multiple cells.

S2, Preprocessing the collected cell information to form structured cell information. The structured cell information includes the number of cells, the number of cell characteristics, and the information for each cell characteristic. At this point, the structured cell information can be viewed as a two-dimensional characteristic matrix, wherein N is the number of cells and P is the number of cell characteristics, with P=1 in this case, meaning the cell characteristic is the magnitude of cellular mechanical forces.

S3, Using the structured cell information as input data to establish a cell characteristic model through supervised, unsupervised, or semi-supervised machine learning, and applying this cell characteristic model for classifying or clustering cells of unknown types or states.

In other embodiments, in the cell identification method, the cellular mechanical force information may also include information on the direction of cellular mechanical forces at a specific point.

In other embodiments, in the cell identification method, the cellular mechanical force information may also include information on changes of the magnitude or direction of cellular mechanical forces at a specific point over a certain time interval.

In some embodiments, in the cell identification method, the cell information may also include cell morphology information.

Nineteenth Embodiment: A Method for Cell Identification (Including Visualization for Qualitative Recognition, as well as Precise Qualitative and Quantitative Recognition)

This embodiment specifically provides a method for recognizing cells using the cellular mechanical force information obtained from any of the system for detecting cellular mechanical force described in the tenth to twelfth embodiments or the cellular mechanical force detection method described in the fourteenth embodiment.

Referring to FIG.s 15,a to g, FIG. 15,a shows the fluorescence imaging of a mixed system of healthy cells and lung non-small cell carcinoma cells; FIG. 15,b displays the distribution of light-reflective signals obtained by the light signal detection device from the device for detecting cellular mechanical force; FIG. 15,c visualizes the magnitude and distribution of cellular mechanical forces; FIG. 15,d is an enlargement image of the representative cellular mechanical force distribution of healthy cells and lung non-small cell carcinoma cells from FIG. 15,c; FIG. 15,e compares the cell morphologies of healthy cells and lung non-small cell carcinoma cells; FIG. 15,f compares the reflection signal intensities (reflecting cellular mechanical forces) of healthy cells, lung non-small cell carcinoma cells, and a mixture of these two cell types in different proportions; FIG. 15,g shows the cluster analysis diagram obtained after processing FIG. 15,c through structured cell information analysis.

Specifically, this embodiment tests healthy cells (Normal) and lung non-small cell carcinoma cell lines (Cancer) using two different fluorescent dyes (Dil& DIO) for pre-staining the cell membranes of healthy and lung cancer cells, which are then mixed in a certain proportion and added to the same device for detecting cellular mechanical force (or to different independent device for detecting cellular mechanical force in other embodiments).

Furthermore, the light signal detection device (a microscope in this embodiment) collects images of the light-reflective signals from the device for detecting cellular mechanical force (as shown in FIG. 15,b), where the high-resolution force field distribution within the two types of cells is directly rendered by the light signal detection device into readable light intensity attenuation signals (reflecting cellular mechanical force intensity) and displayed in the image (as shown in FIG. 15,c). Based on the different degrees of light attenuation displayed in FIG. 15,c for the two types of cells, it is possible to intuitively distinguish between the two cell types through visual observation (qualitative analysis).

Furthermore, the light signal analysis device processes the light-reflective signals from FIG. 15,c. Specifically, this embodiment uses the light signal analysis device (Imaged and Python analysis software in this embodiment, other image analysis software could be used in other embodiments) to collect information from FIG. 15,c on the cellular mechanical force field, including collecting information on the magnitude of cellular mechanical forces at multiple points within each cell, thereby obtaining data on the magnitude of cellular mechanical forces at multiple points across multiple cells; preprocessing the obtained cellular mechanical force magnitude information to form structured cell information; and analyzing the structured cell information to obtain comparative results on cell morphologies between healthy cells and lung non-small cell carcinoma cells (as shown in FIG. 15,e).

The structured cell information includes the number of cells, the number of cell characteristics, and information for each cell characteristic (e.g., cell adhesion area and cell roundness in this embodiment), at this point, the structured cell information can be viewed as a two-dimensional characteristic matrix, where N is the number of cells and P is the number of cell characteristics, with P=2 in this case, meaning the cell characteristics are: the magnitude of cellular mechanical forces and the distribution of cellular mechanical forces within the cell.

Furthermore, using the above-structured cell information as input data, supervised machine learning is employed to establish a cell characteristic model (comparing pre-staining of the two cell lines with different cell membrane dyes (Dil& DIO)), and using the structured cell information of a large number of cells to train the cell characteristic model, resulting in the cluster analysis diagram shown in FIG. 15,g. The obtained cell characteristic model is then applied for classifying and identifying cells of unknown types or states. It can be seen that based on structured cell characteristic data (the magnitude of cellular mechanical forces and the distribution of cellular mechanical forces within the cell) as input data, it is possible to use the light signal analysis device (Imaged and Python analysis software in this embodiment, other cluster analysis software in other embodiments) to cluster and type normal healthy cells and cancer cells, thereby achieving recognition of unknown cell types.

FIG. 15,e shows no statistically significant difference in the morphology (including cell adhesion area and cell roundness) among different cells; FIG. 15,f demonstrates the significant difference in reflection signal intensity (reflecting cellular mechanical force) between normal and tumor cells, as well as a certain linear relationship between reflection signal intensity and the mixing ratio when normal and tumor cells are mixed together in certain proportions. It can be seen that, compared to other cell characteristics (e.g., cell adhesion area, cell roundness, and other morphological information in FIG. 15,e), the cellular mechanical characteristics measured by the device for detecting cellular mechanical force of this invention allow for a more intuitive and precise recognition of cell status and type (both quantitative and qualitative analysis).

Additionally, data from FIG.s 15,d and f indicate that tumor cells exhibit higher cellular mechanical force magnitude and more uneven distribution than normal cells. It is evident that after visualizing cellular mechanics in image form, it is possible to visually distinguish the force field characteristics of different cells with the naked eye; and further, by structurally processing the force field magnitude of different cells through image analysis software, comprehensive analysis yields the cell morphology information in FIG. 15,e, the reflection signal intensity (reflecting cellular mechanical) in FIG. 15,f, and the cluster analysis diagram in FIG. 15,g. This invention, through comprehensive analysis of the structured force field information of different cells (e.g., healthy cells and non-small cell lung cancer cells in this embodiment), can cluster and quantitatively analyze different cells, thereby achieving precise recognition of cell types.

In summary, the device for detecting cellular mechanical force of this invention not only allows for qualitative analysis through visual distinction but also enables more intuitive and precise recognition of cell status and type based on the measured cellular mechanical characteristics (both quantitative and qualitative analysis), confirming that using the cellular mechanical force field as a biomarker can better distinguish the cell types.

Twentieth Embodiment: A Method for Detecting Cell Viability

This embodiment specifically provides using the cellular mechanical force information obtained from any of the system for detecting cellular mechanical force described in the tenth to twelfth embodiments or the cellular mechanical force detection method described in the fourteenth embodiment to monitor cell viability.

Referring to FIG.s 16,a-c, wherein FIG. 16,a shows a schematic diagram of the operational procedure of the cell viability detection method; FIG. 16,b compares the cell viabilities of A549 cells treated with different doses of 5FU for 24 hours, measured by the MTT assay and the cellular mechanical force determined by the device, system, or method of this invention; FIG. 16,c compares cell viabilities and cellular mechanical forces of A549 cells treated with different doses of 5FU for different time, measured by the MTT assay and the device, or the system, or the method in this invention, separately.

Specifically, this embodiment cultures non-small cell lung cancer cells A549 on multiple device for detecting cellular mechanical force, treats them with different doses of the cell proliferation inhibitor 5-fluorouracil (5-FU), and monitors cellular mechanical forces at different time points using the system for detecting cellular mechanical force or the method described in the tenth to twelfth embodiments or the fourteenth embodiment. Cell proliferation and cytotoxicity are monitored at different time points using the CCK-8 assay kit, with cell viability measured by the MTT assay serving as a control, generating the data for FIG.s 16,b and c.

FIG.s 16,b and c demonstrate that both cell viability measured by the conventional MTT assay and cell viability reflected by cellular mechanical forces show a dose-dependent decline, indicating a positive correlation between cellular mechanical force and cell viability.

Moreover, as shown in FIG. 16,b, compared to the control group (DMSO), cellular viability reflected by cellular mechanical force shows a greater reduction after treatment with different doses of 5FU for 24 hours, allowing for a more intuitive assessment of cell viability. As shown in FIG. 16,c, while the MTT assay shows no significant change in cell viability within 12 hours of 5FU treatment. Measuring cellular mechanical force can detect a decrease in cellular mechanical force at an earlier time point before the MTT assay detects a decrease in cellular metabolic activity. Specifically, a significant decrease trend is observed at 6 hours with a 0.5 $\mu$M treatment dose, and at 3 hours with a 1 $\mu$M treatment dose, thereby characterizing the decrease in cell viability more sensitively.

In summary, this embodiment demonstrates that directly detecting cellular mechanical force using the device for detecting cellular mechanical force is a highly sensitive and effective method for evaluating cell response to drug treatment in terms of viability.

Twenty-First Embodiment: A Method for Detecting Cell State

This embodiment specifically provides using the cellular mechanical force information obtained from the system for detecting cellular mechanical force described in the twelfth embodiment or the cellular mechanical force detection method described in the fourteenth embodiment to analyze cell states.

Referring to FIG.s 17,a to f, where FIG. 17,a illustrates the operational process of the cell state detection method; FIG.s 17,b and c show fluorescence microscopy images of macrophages differentiating into M1 and M2 states, respectively; FIG. 17,d compares the cell adhesion area of M0 macrophages, M1, and M2 states; FIG. 17,e compares the roundness of cells in M0, M1, and M2 states; FIG. 17,f compares the cellular mechanical forces of cells in M0, M1, and M2 states.

Specifically, this embodiment uses macrophages as test subjects, cultures them on different independent device for detecting cellular mechanical force, and uses lipopolysaccharide (LPS) and interleukin IL4 to induce differentiation from M0 to M1 and M2 states, respectively, with M0 serving as the control. After differentiation, images are captured using a light signal detection device (a microscope in this embodiment) for FIG.s 17, b and c, and further image processing and data analysis are conducted using light signal analysis software (Imaged and Python) to convert into structured information and then analyze to produce the data for FIG.s 17,d-f. From FIG.s 17,a to f, both from direct observation (FIG.s 17,b and c) and structured data quantification (FIG.s 17, d-f), clear differences are observed between M0 macrophages and their differentiated M1 and M2 states.

Twenty-Second Embodiment: A Method for Detecting Multicellular Aggregates

This embodiment specifically provides using the cellular mechanical force information obtained from the system for detecting cellular mechanical force described in the twelfth embodiment or the cellular mechanical force detection method described in the fourteenth embodiment to analyze cell states.

Specifically, this embodiment provides for the cultivation of multicellular aggregates on the device for detecting cellular mechanical force in various ways, including two specific methods of combination:
The first method involves setting culture medium on the micropillars of the device for detecting cellular mechanical force, transplanting cells into the culture medium on the micropillars to cultivate multicellular aggregates. In some embodiments, this method allows for real-time monitoring of cell culture processes, applicable to studying the effects of chemical, biological, and physical external stimuli on cell growth, such as culture media and drugs.

The second method directly attaches cultured multicellular aggregates to the micropillars of the device for detecting cellular mechanical force for detection.

More specifically, this embodiment provides a method for culturing a tumor cell multicellular aggregate on the device for detecting cellular mechanical force for drug sensitivity test, including the steps:

S1, Generation of tumor cell multicellular aggregates: FN 50 $\mu$g/mL is applied to the top of the micropillars of the device for detecting cellular mechanical force and sterilized with UV for 30 minutes; breast cancer cells MCF-7 (approximately $1\times$ to $9\times$ cells) are seeded on the top surface of the micropillars (number of micropillars is not limited) of the device for detecting cellular mechanical force, which is then immersed in 3dGRO™ Spheroid Medium (S3077) for more than 3 days to induce the formation of tumor cell multicellular aggregates.

S2, Applying the generated tumor cell multicellular aggregates to a 5-Fu drug sensitivity test: 200 $\mu$M of 5-Fu is added to the tumor cell multicellular aggregates and cultured for one day; in the test, before the addition of 5-Fu and and one day of culture after the addition of 5-Fu on the device for detecting cellular mechanical force (with tumor cell multicellular aggregates, including the culture medium), light-reflective signals are captured by the light signal detection device (CCD sensor) and cellular mechanical force distribution images are obtained using the light signal analysis device (ImageJ), as shown in FIG. 18.

Referring to FIG.s 18,a-b, where FIG. 18,a shows characterization images of tumor cell multicellular aggregates with/without 5-Fu treatment for the first morphology; FIG. 18,b for the second morphology; from left to right are the mixed images of cell membrane fluorescence and reflection signals (1), light-reflective signal (2), cell nucleus (3), cell membrane (4), and the cellular mechanical force visualization image processed by the light signal analysis device (ImageJ) (5). Due to cell heterogeneity, tumor cells differ, and multicellular aggregates exhibit various morphologies, hence, they adhere together in different forms. This embodiment selects two representative morphologies for cell morphology detection, the first morphology refers to the cell morphology in which two larger cells adhered together, and the second morphology refers to the cell morphology of a group of small cells adhered together.

FIG.s 18,a-b show that after treating cells with anti-tumor drugs, the cell viability is reduced, and the reflection signals noticeably weaken; moreover, the changes in cellular mechanical forces before and after 5-Fu treatment reveal significant differences in the drug sensitivity between the two different morphologies of tumor cell multicellular aggregates.It is evident that the device for detecting cellular mechanical force of this invention can perform cellular mechanical force measurements on multicellular aggregates (such as tumor multicellular aggregates) and can use cellular mechanical force to monitor the vitality state of cell aggregates and distinguish different cell morphologies.

In the definition of this invention, a multicellular aggregate refers to:
Cells are the basic structural and functional units of organisms, usually proliferating or differentiating to form groups of two or more cells, i.e., multicellular aggregates; multicellular aggregates include tumor multicellular aggregates and other cell groups obtained through in vitro or in vivo culture.

In summary, the cell detection device of this invention for detecting cellular mechanical force, cell identification, cell state detection, and cell viability detection offers high sensitivity and effectiveness, and allows for real-time monitoring, applicable to studying cell responses to drug treatments.

Distinct from existing technologies, the technical solutions of this invention offer the following advantages:

Firstly, high-throughput and low cost: Compared to existing TFM and ordinary arrays of micropillars, the technical solutions of this invention eliminate dependence on microscopes, significantly simplifying the operational process since high-resolution imaging through microscopes is not required, and high-throughput cell monitoring can be achieved simply by monitoring the intensity of reflected light.
Secondly, single cell resolution: with high resolution, it allows for real-time monitoring of each cell and can be combined with other single cell analysis techniques to measure heterogeneity in cell responses to drugs; real-time monitoring without the need for fluorescence, thus avoiding phototoxic effects of lasers on cells, making it suitable for long-term monitoring to study long-term cell responses to drugs; high sensitivity through signal amplification of micropillar deformation, enhancing sensitivity to deformation monitoring. Detection of micro and nano-pillar bending deformation generally relies on optical systems (such as microscopes), but the smaller the size of the micropillars is, the higher the precision and resolution requirements for the optical system are to be needed. For instance, micropillars that are 2 microns wide and 6 microns tall require an objective lens of more than 20x in combination with a confocal system for effective observation. However, this invention utilizes the principle of specular reflection to detect the attenuation of reflected light, effectively amplifying the signal of micropillar deformation, which has been experimentally verified to be observable even under a 5x objective lens. With a special reading system, it is possible to effectively detect micropillar deformation without relying on high-magnification optical objective lenses, thereby greatly reducing system costs and effectively increasing throughput.

Lastly, the capability to simulate the cellular microenvironment; it can simulate the components and morphology of the extracellular matrix, meeting a richer range of technical demands.

It should be noted that although the above embodiments have been described, they do not limit the scope of patent protection for this invention. Therefore, changes and modifications made to the embodiments described herein based on the innovative concept of this invention, or the equivalent structure or equivalent process transformations made using the content of this invention's specification and drawings, whether applied directly or indirectly in other relevant technical fields, are included within the scope of patent protection for this invention.

## Claims

1. A device for detecting cellular mechanical force, **characterized by** comprising:

   a base; and
   an array of micropillars located on the base, the micropillars being deformable in respond to a cellular mechanical force, and a light-reflective layer located on the top or an upper portion of the micropillars.

2. The device according to claim 1, wherein the base is light transmissive, the top of the micropillars has the light-reflective layer, and the bodies of the micropillars are capable of transmitting light.

3. The device according to claim 2, wherein the surfaces of the micropillars comprises an anti-reflection layer.

4. The device according to claim 1, wherein the light reflective layer is made from a material selected from a group consisting of Metal foil layer, metal oxides or metal salts, ultrafine glass beads, micro-prisms, organic reflective materials, or a combination thereof.

5. The device according to claims 1 to 4, wherein all or some of the micropillars have a cell adhesion substance coated at a top surface thereof.

6. The device according to claim 5, wherein the cell adhesion substance is one or more of the following substances: an extracellular matrix molecule selected from a group consisting of collagen, fibronectin, vitronectin, laminin or proelastin; an extracellular matrix mimic substance including polypeptides containing RGD adhesion sequences; a substance with cell adhesion promotion mechanism including polylysine; or a substance interactable with cell surface receptors.

7. The device according to claim 5, wherein some of the micropillars having the cell adhesion substance on the top surface thereof form a preset pattern.

8. The device according to claim 5, wherein some micropillarsin a preset areas of the micropillars having the cell adhesion inhibiting substance on the top surfaces.

9. The device according to claim 1, wherein a cross-sectional shape of the micropillars is circular, elliptical or polygonal.

10. The device according to claim 1, wherein dimensions of micropillars and the array of micropillars includes heights of the micropillars in a range of from 10nm to 500μm, inter-pillar distances in a range of 10nm to 50μm, and diameters at the top surface of the micropillars in a range of 50nm to 50μm.

11. The device according to claim 1, further comprising a cell restraining mechanism wherein the cell restraining mechanism comprises one or more restraining surfaces, the one or more restraining surfaces beingare flat surface or curved surface, and beingare perpendicular to the base, wherein the restraining surface has a height greater than the micropillars and enclosures a preset number of the micropillars.

12. A system for detecting cellular mechanical force, **characterized by** comprising:
a device for detecting cellular mechanical force as defined in any one of claims 1-11;a light signal generation device having a light source, wherein the light emitted by the light source being illuminated to the light-reflective layer of the micropillars through an incident light path; and a light signal detection device being configured for detecting light reflected from the light-reflective layer of the micropillars, wherein the light reflected by the light-reflective layer enters the light signal detection device through a light-reflective path.

13. The system according to claim 12, wherein the base of the cellular mechanical force detection device is light transmissive, and the body of the micropillars are capable of transmitting light; the top of the micropillars has the light-reflective layer; wherein the light emitted by the light source is illuminated from the baseto the light-reflective layer of the micropillars through an incident light path; and the light signal detection device is configured for detecting the light reflected from the light-reflective layer.

14. The system according to claim 12, further comprising a light signal analysis device for analyzing the light signal.

15. A method for detecting cellular mechanical force, **characterized by** comprising:

    emitting light by using the light signal generation device as defined in any one of claims 12 to 14 ; and
    detecting the reflected light by using the light signal detection device as defined in any one of claims 12 to 14 to detect the reflected light after the light has interacted with the device for detecting cellular mechanical force.

16. The method according to claim 15, further comprising using a light signal analysis device to compare and analyze the reflected light before interactions through the cell and device and after interactions through the cell and device to obtain cellular mechanical force information including a magnitude, direction, or frequency of the cellular mechanical forces.

17. A method for detecting cell status, **characterized by** comprising: obtaining cellular mechanical force information through a system for detecting cellular mechanical forces as described in any one of claims 10-12, or through a method for detecting cellular mechanical forces as described in any one of claims 15-16, and determining the cell status based on the cellular mechanical force information.
    wherein the cell status includes cell adhesion, viability, differentiation/activation, proliferation, and/or migration.

18. A method for cell identification, comprising: obtaining cellular mechanical force information through a system for detecting cellular mechanical forces as described in any one of claims 12-14, or through a method for detecting cellular mechanical forces as described in any one of claims 15-16, and distinguishing cell types based on the cellular mechanical force information.

19. A method for preparing a cell mechanical force detection device, comprising the following steps:laying a layer of reflective material on the top or upper half surface of the micropillars to obtain micropillars with reflective layers on the top or upper half surface.

20. The method according to claim 19, further comprising the step before "applying a reflective layer on the top or upper half of the micropillars":

    coating the entire micropillars with an anti-reflective layer uniformly;
    removing the anti-reflective layer from the top or upper half surfaces of the micropillars.

21. The method according to claim 19 or 20, **characterized in that** the specific process of "laying a layer of reflective material on the top or upper half surface of the micropillars to obtain micropillars with reflective layers on the top or upper half surface" is as follows: uniformly sputtering a layer of reflective metal on the top or upper half surface of the micropillars to obtain micropillars with metal reflective layers on the top or upper half surface.

FIG. 1

a)

b)

FIG. 2

FIG. 3

FIG. 4

FIG. 5

FIG. 6

FIG. 7

recessed space 15

micropillar 12

base 11

restraining surface 16

FIG. 8

restraining surface 16

micropillar 12

base 11

FIG. 9

a)

Microwell

b)

A549 cell membrane (Dil)
Light Reflection

c)

FIG. 10

FIG. 11

a)

Reflective Layer (metal)    Cell

Illumination    Reflection

LIGHT SIGNAL GENERATION DEVICE

LIGHT SIGNAL DETECTION DEVICE

LIGHT SIGNAL ANALYSIS DEVICE

b)

c)

Bright field reflection (with cell): I

FFT generated reflection (no cell): $I_0$

$I_0$ subtract with I

Applied with force index: j

FIG. 12

FIG. 13

FIG. 14

a) Lung cancer cell / Normal cell

b)

c) Mechanical Force (nN) (Traction force)

d) Normal / Cancer

e) Cell area (µm²) — Normal, Cancer (ns); Cell circularity — Normal, Cancer (ns)

f) Reflective Intensity — Normal, Cancer, Mix1, Mix2
Mix1: 90% Normal + 10% Cancer
Mix2: 80% Normal + 20% Cancer

g) Cancer / Normal — PC1, PC2

FIG. 15

FIG. 16

FIG. 17

FIG. 18

# INTERNATIONAL SEARCH REPORT

| International application No. |
| --- |
| **PCT/CN2022/121338** |

| A. | CLASSIFICATION OF SUBJECT MATTER |
| --- | --- |
| | G01N 33/483(2006.01)i; G01N 21/55(2014.01)i |

According to International Patent Classification (IPC) or to both national classification and IPC

| B. | FIELDS SEARCHED |
| --- | --- |

Minimum documentation searched (classification system followed by classification symbols)

G01N

Documentation searched other than minimum documentation to the extent that such documents are included in the fields searched

Electronic data base consulted during the international search (name of data base and, where practicable, search terms used)

CNTXT; ENTXTC; VEN; DWPI; ENTXT: 细胞, 力, 机械力, 牵引力, 微, 柱, 反射, 弯, 曲, 形变, 变形, cell, force?, mechanical, traction, physical, micropillar?, reflect+, curv+, deform+

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | US 2015300953 A1 (THE UNIVERSITY OF NORTH CAROLINA AT CHAPEL HILL) 22 October 2015 (2015-10-22) description, paragraphs 15-63, and figure 1 | 1-21 |
| Y | US 2018372635 A1 (THE REGENTS OF THE UNIVERSITY OF CALIFORNIA) 27 December 2018 (2018-12-27) description, paragraphs 31-99, and figure 1 | 1-21 |
| Y | US 2020116696 A1 (THE REGENTS OF THE UNIVERSITY OF CALIFORNIA) 16 April 2020 (2020-04-16) description, paragraphs 11-117, and figure 3 | 1-21 |
| Y | CN 109827928 A (SOUTHEAST UNIVERSITY) 31 May 2019 (2019-05-31) description, paragraphs 9-23 | 1-21 |
| Y | CN 111812095 A (SUZHOU MEDICAL EQUIPMENT RESEARCH INSTITUTE OF SOUTHEAST UNIVERSITY) 23 October 2020 (2020-10-23) description, paragraphs 18-31 | 1-21 |
| Y | CN 106338500 A (BEIJING INSTITUTE OF NANOENERGY AND NANOSYSTEMS) 18 January 2017 (2017-01-18) description, paragraphs 20-33, and figures 1-2 | 1-21 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **19 November 2022** | **16 December 2022** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2022/121338**

### C.    DOCUMENTS CONSIDERED TO BE RELEVANT

| Category* | Citation of document, with indication, where appropriate, of the relevant passages | Relevant to claim No. |
|---|---|---|
| A | US 2014024045 A1 (MANUEL, T. et al.) 23 January 2014 (2014-01-23)<br>entire document | 1-21 |
| A | US 2010098941 A1 (KOREA INSTITUTE OF SCIENCE AND TECHNOLOGY) 22 April 2010 (2010-04-22)<br>entire document | 1-21 |
| A | JP 2008055556 A (NATIONAL INSTITUTE FOR MATERIALS SCIENCE et al.) 13 March 2008 (2008-03-13)<br>entire document | 1-21 |
| A | US 2015093823 A1 (HARVARD COLLEGE) 02 April 2015 (2015-04-02)<br>entire document | 1-21 |

Form PCT/ISA/210 (second sheet) (January 2015)

**INTERNATIONAL SEARCH REPORT**
Information on patent family members

International application No.

**PCT/CN2022/121338**

| Patent document cited in search report | | | Publication date (day/month/year) | Patent family member(s) | | | Publication date (day/month/year) |
|---|---|---|---|---|---|---|---|
| US | 2015300953 | A1 | 22 October 2015 | WO | 2014085804 | A1 | 05 June 2014 |
| | | | | EP | 2926115 | A1 | 07 October 2015 |
| | | | | US | 9952149 | B2 | 24 April 2018 |
| | | | | EP | 2926115 | B1 | 26 May 2021 |
| US | 2018372635 | A1 | 27 December 2018 | WO | 2017015063 | A1 | 26 January 2017 |
| | | | | US | 10712271 | B2 | 14 July 2020 |
| US | 2020116696 | A1 | 16 April 2020 | WO | 2019010234 | A1 | 10 January 2019 |
| CN | 109827928 | A | 31 May 2019 | WO | 2020155716 | A1 | 06 August 2020 |
| | | | | WO | 2020155324 | A1 | 06 August 2020 |
| | | | | CN | 109827928 | B | 11 October 2019 |
| CN | 111812095 | A | 23 October 2020 | CN | 111812095 | B | 25 December 2020 |
| | | | | WO | 2022052361 | A1 | 17 March 2022 |
| CN | 106338500 | A | 18 January 2017 | WO | 2017008699 | A1 | 19 January 2017 |
| | | | | CN | 106338500 | B | 01 November 2019 |
| US | 2014024045 | A1 | 23 January 2014 | ES | 2538985 | T3 | 25 June 2015 |
| | | | | WO | 2012107383 | A1 | 16 August 2012 |
| | | | | EP | 2485050 | A1 | 08 August 2012 |
| | | | | EP | 2485050 | B1 | 01 April 2015 |
| | | | | US | 9250241 | B2 | 02 February 2016 |
| US | 2010098941 | A1 | 22 April 2010 | KR | 20100042436 | A | 26 April 2010 |
| | | | | EP | 2177558 | A1 | 21 April 2010 |
| | | | | KR | 101027012 | B1 | 11 April 2011 |
| | | | | KR | 1EP2177558 | B1 | 28 September 2016 |
| JP | 2008055556 | A | 13 March 2008 | JP | 5190571 | B2 | 24 April 2013 |
| US | 2015093823 | A1 | 02 April 2015 | None | | | |

Form PCT/ISA/210 (patent family annex) (January 2015)